# EUROPEAN PATENT APPLICATION

(11) **EP 4 101 490 A1**
(43) Date of publication of application: **14.12.2022**
(21) Application number: 21382519.3
(22) Date of filing: 11.06.2021
(51) Int. Cl.: A61M 25/00

(54) **CATHETER FOR CEREBRAL ANGIOGRAPHY AND NEUROINTERVENTIONS**

(71) Applicant: Barranco Pons, Roger, 08640 Olesa de Montserrat (ES)
(72) Inventor: Barranco Pons, Roger, 08640 Olesa de Montserrat (ES)
(74) Representative: Juncosa Miró, Jaime

(57) **Abstract**

A catheter for cerebral angiography and neurointerventions is disclosed. The catheter comprises a proximal end, a distal end and a plurality of segments. The plurality of segments include: a first curved segment having a first radius of curvature and being extended with a first linear segment; a second curved segment connecting the first linear segment with a second linear segment, the second curved segment having a second radius of curvature, wherein a concave portion of the second curved segment faces an area delimited by the first linear segment and the second linear segment and wherein the length of the second linear segment is greater than the length of the first linear segment; and a third linear segment that is connected to an end of the second linear segment through a curved portion that orients the third linear segment in a direction towards the first curved segment.

## Description

### Technical Field

The present invention is directed, in general, to the field of catheters. In particular, the invention relates to a catheter for cerebral angiography and neurointerventions.

### Background of the Invention

Transfemoral access (TFA) has been widely used in neurointerventional procedures; it is the vascular approach most frequently used for catheterization of the supraaortic and intracranial vessels. However, TFA can lead to potentially life-threatening complications, which has sparked interest in transradial access (TRA) as a safer access option.

In a review of 19,826 consecutive patients undergoing diagnostic cerebral angiography using TFA, access-site hematoma was the most common complication overall (4.2%).

Percutaneous radial access was described by Campeau in 1989 for coronary angiography in 100 patients. Since then, a growing body of evidence suggests that TRA is safer for patients and more cost-effective.

In the 2018 ESC/EACTS guidelines on myocardial revascularization, radial access is preferred for any PCI irrespective of clinical presentation, unless there are overriding procedural considerations.

During all this years, technical innovations and specific TRA catheters for gaining access to the coronary arteries have helped to change the TFA to TRA in the cardiology field. Likewise, TRA access is showing to be a good choice for elderly patients to perform stroke thrombectomy or other kinds of neuro vascular diseases.

Though TRA has become progressively accepted for diagnostic cerebral angiography and neurointerventional procedures, there is still a need for proper specific materials to perform neurointerventions through TRA.

The known standard catheters in the field, such as Cobra catheters or Simmons catheters when used in the left vertebral artery show difficulties in its manipulation, not being able to rise in the left vertebral artery but tending to fall into the descending aorta. This also may happen with the other supraaortic vessels depending on the anatomy of the aortic arch.

Besides, International patent application WO2020072895 discloses a cardiac catheter for angiographic procedures of a left internal mammary artery bypass graft performed using a right radial arterial approach. The catheter includes a guide catheter comprising an elongated member having an outer sheath body portion that includes (see Figs. 3A and 3C) an outer sheath proximal end, an outer sheath distal end, and an outer sheath lumen extending through the outer sheath between the outer sheath proximal end and the outer sheath distal end. The outer sheath includes a curved section that has: a first curved segment, a second curved segment, a third curved segment, a fourth curved segment, a first extended segment between the third curved segment and the fourth curved segment, and a second extended segment between the fourth curved segment and the outer sheath distal end.

More specific catheters to perform supraaortic vessels catheterization from a TRA access are therefore needed.

### Description of the Invention

To that end the present invention provides a catheter for cerebral angiography and neurointerventions, comprising: a proximal end, a distal end and a plurality of segments. The plurality of segments include a first curved segment, a second curved segment and a third linear segment.

The firs curved segment has a first radius of curvature and is extended with a first linear segment. The second curved segment connects the first linear segment with a second linear segment and has a second radius of curvature. A concave portion of the second curved segment faces an area delimited by the first linear segment and the second linear segment. The second linear segment has a length greater than a length of the first linear segment.

Unlike the known catheter devices in the field, in the proposed catheter the third linear segment is connected to an end of the second linear segment through a curved portion that orients the third linear segment in a direction towards the first curved segment.

In some embodiments, the curved portion comprises a radius of curvature that is smaller than the first radius of curvature.

In some embodiments, the second linear segment and the first linear segment are approximately parallel one to another.

In some embodiments, the intersection of the third linear segment and the second linear segment covers an angle of at least 160º.

Particularly, the first radius of curvature is smaller than the second radius of curvature.

The proposed catheter can have different lengths. Particularly, the total length of the catheter is between 80 cm and 150 cm. Moreover, in some embodiments, the second linear segment can have a length comprised in a range between 3 - 10 cm and the third linear segment can have a length comprised in a range between 70-147 cm.

The proposed catheter device enables going up in the left vertebral artery without dropping into the descending aorta while offering an improved handling and control. Likewise, the catheter provides a good torque and pushability combination.

The foregoing describes embodiments of the present invention and modifications, obvious to those skilled in the art can be made thereto, without departing from the scope of the present invention.

### Brief Description of the Drawings

The previous and other advantages and features will be more fully understood from the following detailed description of embodiments, with reference to the attached figures, which must be considered in an illustrative and non-limiting manner, in which:

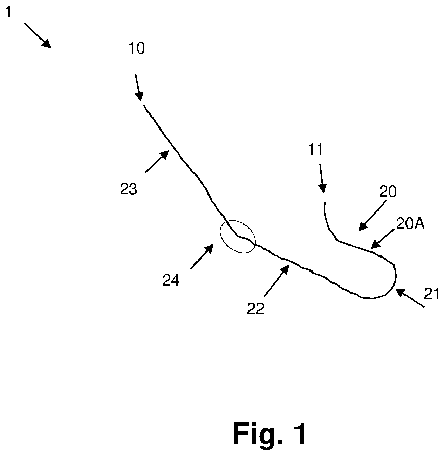

Fig. 1 schematically illustrates a catheter for cerebral angiography and neurointerventions, according to an embodiment of the present invention.

### Detailed Description of Preferred Embodiments

Fig.1 shows a schematic illustration of the proposed catheter 1. Particularly, the catheter 1 is used for cerebral angiography and neurointerventions.

According to the embodiment of Fig. 1, the catheter 1 has a proximal end 11, a distal end 10 and a series of segments 20, 21, 22, 23 connecting said proximal end 11 and distal end 10. Although not illustrated in the figure, in some embodiments, the proposed catheter 1 also comprises an outer sheath, for example a 2.7-2.8 outer diameter for 6F sheath, among others.

As shown in the figure, a first curved segment 20 comprises a curve with a first radius of curvature and extends with a first linear segment 20A. A second curved segment 21 extends from the first segment 20 and has a curve with a second radius of curvature. The second curved segment 21 connects the first linear segment 20A with a second linear segment 22. The second linear segment has a length which is larger than a length of the first linear segment 20A. A third linear segment 23 is connected to an end of the second linear segment 22. Particularly, the connection is done by means of a curved portion 24 that purposely orients the third linear segment 23 in a direction that approaches towards the first curved segment 20. The radius of curvature of the curved portion 24 is particularly smaller than the first radius of curvature.

In some embodiments, the intersection of the third linear segment 23 and the second linear segment 22 covers an angle of at least 160º.

As shown in the illustrated embodiment, the second linear segment 22 and the first linear segment 20A are approximately parallel one to another. Likewise, the second radius of curvature is greater than the first radius of curvature.

In various embodiments, the overall length of the catheter 1 can be between 80 and 150 cm. The length of the second linear segment 22 can be between 3 and 10 cm. The length of the third linear segment 23 can be between 70 and 147 cm.

In some embodiments, the distal 10 or proximal 11 ends of the catheter 1, or other segments thereof, can be provided with a radiopaque element to enable visualization under fluoroscopy, or other imaging technique.

Unless otherwise indicated, all numbers expressing measurements, conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed patient matter.

As used herein, the term "about", when referring to a value or to an amount of a length, percentage, etc., is meant to encompass variations of in some embodiments ±10%, in some embodiments ±5%, in some embodiments ±1%, in some embodiments ±0.5%, and in some embodiments ±0.1% from the specified amount, as such variations are appropriate to perform the disclosed device.

Without further elaboration, it is believed that one skilled in the art can use the preceding description to utilize the invention to its fullest extent. The claims and embodiments disclosed herein are to be construed as merely illustrative and exemplary, and not a limitation of the scope of the present disclosure in any way. It will be apparent to those having ordinary skill in the art, with the aid of the present disclosure, that changes may be made to the details of the above-described embodiments without departing from the underlying principles of the disclosure herein. In other words, various modifications and improvements of the embodiments specifically disclosed in the description above are within the scope of the appended claims. The scope of the invention is therefore defined by the following claims and their equivalents.

## Claims

1. A catheter for cerebral angiography and neurointerventions, comprising: a proximal end (11), a distal end (10) and a plurality of segments (20, 21, 22, 23), the plurality of segments including:
a first curved segment (20) having a first radius of curvature, the first curved segment (20) being extended with a first linear segment (20A);
a second curved segment (21) connecting the first linear segment (20A) with a second linear segment (22), the second curved segment (21) having a second radius of curvature, wherein a concave portion of the second curved segment (21) faces an area delimited by the first linear segment (20A) and the second linear segment (22);
the second linear segment (22) has a length greater than a length of the first linear segment (20A);
**characterized in that:**
the plurality of segments (20, 21, 22, 23) further comprises a third linear segment (23) that is connected to an end of the second linear segment (22) through a curved portion (24) that orients the third linear segment (23) in a direction towards the first curved segment (20).

2. The catheter of claim 1, wherein the curved portion (24) comprises a radius of curvature that is smaller than the first radius of curvature.

3. The catheter of claim 1 or 2, wherein the second linear segment (22) and the first linear segment (20A) are approximately parallel one to another.

4. The catheter of any one of the previous claims, wherein an intersection of the third linear segment (23) and the second linear segment (22) covers an angle of at least 160º.

5. The catheter of any one of the previous claims, wherein the first radius of curvature is smaller than the second radius of curvature.

6. The catheter of any one of the previous claims, wherein a total length of the catheter is between 80 cm and 150 cm.

7. The catheter of any one of the previous claims, wherein the second linear segment (22) has a length comprised in a range between 3 - 10 cm and the third linear segment (23) has a length comprised in a range between 70-147 cm.
